Europäisches Patentamt

European Patent Office

Office européen des brevets

(19)

(11) Numéro de publication: **0 251 941**
**A1**

## (12) DEMANDE DE BREVET EUROPEEN

(21) Numéro de dépôt: 87420140.3

(22) Date de dépôt: 20.05.87

(51) Int. Cl.⁴: **C 07 C 79/26**
**C 07 C 69/96**

(30) Priorité: 29.05.86 FR 8607918

(43) Date de publication de la demande:
07.01.88 Bulletin 88/01

(84) Etats contractants désignés:
AT BE CH DE ES FR GB GR IT LI LU NL SE

(71) Demandeur: RHONE-POULENC CHIMIE
25, quai Paul Doumer
F-92408 Courbevoie Cédex (FR)

(72) Inventeur: Desbois, Michel
526, Chemin du Bois
F-69140 Rilleux la Pape (FR)

(74) Mandataire: Cazes, Jean-Marie et al
RHONE-POULENC INTERSERVICES Service Brevets
Chimie 25, quai Paul Doumer
F-92408 Courbevoie Cédex (FR)

(54) Procédé de préparation du paranitrophénol.

(57) La présente invention concerne un nouveau procédé de préparation du paranitrophénol par mise en contact dans l'acide fluorhydrique liquide d'un ester minéral de phényle avec un agent de nitration et en ce qu'on réalise simultanément ou successivement une hydrolyse du produit obtenu.

EP 0 251 941 A1

**Description**

PROCEDE DE PREPARATION DU PARANITROPHENOL

La présente invention concerne un procédé de préparation du paranitrophénol. Elle concerne plus particulièrement un procédé de préparation du paranitrophénol à partir d'esters minéraux de phényle.

La nitration directe du phénol est industriellement non rentable car on obtient um mélange d'isomères ortho et para où l'isomère ortho est prédominant et difficilement séparable de l'isomère para. Pour éviter cet inconvénient, divers procédés ont été proposés notamment des procédés partant de carbonate de diphényle qui est nitré puis hydrolysé pour donner le paranitrophénol.

Ainsi il a aussi été proposé dans le brevet allemand publié sous le no 2 557 6l4 un procédé de nitration directe de carbonate de biphényle caractérisé par le fait que la concentration de l'acide nitrique mis à l'origine dans le milieu réactionnel est inférieure à 85 %, et la concentration de l'acide nitrique introduit ensuite est supérieure à 95%. Les rendements en carbonate de bis (nitro-4 phényle) ne dépassent jamais 78 % car la répartition isomérique entre les dérivés ortho et para substitués n'est pas optimale et ne dépasse jamais 80 % en isomère para.

Il est également connu d'après le brevet français no 76/33l48 de procéder à la nitration de carbonate de diphényle dans l'acide sulfurique concentré. Ce procédé permet d'obtenir des rendements d'environ 80 % en carbonate de bis (nitro-4 phényle) la répartition isomérique entre les dérivés ortho et para substitué étant meilleure que dans le brevet précédent.

Les procédés utilisant l'acide sulfurique comme solvant présentent cependant quelques inconvénients car l'acide sulfurique est un composé très oxydant, dont la viscosité est élevée ce qui entraîne pour l'utilisateur des problèmes d'agitation. D'autre part le fait que l'acide sulfurique ait un point d'ébullition élevé empêche un recyclage aisé de ce dernier et par conséquent entraîne alors une élimination difficile des effluents que sont les sulfates.

La présente invention a permis d'éviter l'emploi d'acide sulfurique et donc les problèmes de nuisance liés à son utilisation. Elle concerne un procédé de préparation de paranitrophénol, caractérisé en ce qu'on met en présence dans l'acide fluorhydrique liquide un ester minéral de phényle avec un agent de nitration et en ce qu'on réalise simultanément ou successivement une hydrolyse du produit obtenu.

Les esters minéraux de phényle pouvant être mis en oeuvre dans le cadre de la présente invention sont choisis parmi les borates de phényle, les phosphates de phényle, les sulfates de phényle, les carbonates de phényle, l'arseniate de phényle, les antimoniates de phényle, l'orthosilicate de phényle, le séléniate de monophényle.

On préfère cependant utiliser le carbonate de diphényle ou le phosphate de triphényle.

L'agent de nitration peut être choisi parmi les acides nitriques disponibles couramment tels que l'acide nitrique fumant, l'acide nitrique à 65 %, les sels alcalins de l'acide nitrique tels que notamment les nitrates de sodium ou de potassium et les sels de nitronium tels que le tétrafluoroborate de nitronium. On préfère tout particulièrement utiliser les acides nitriques du commerce présentant une concentration supérieure à 65 %.

L'acide fluorhydrique utilisé comme solvant est de préférence anhydre bien que l'eau ne soit pas néfaste à la réaction car elle est déjà apportée en partie par l'acide nitrique.

Des solvants inertes autre que l'acide fluorhydrique peuvent, être utilisés, dans le cas où l'on part de matières premières très fragiles telles que notamment le phosphate de triphényle. Parmi les solvants utilisables on peut citer le chlorure de méthylène, le tétrachlorure de carbone, le trifluoro l,l,2-trichloro l,2,2-éthane, le trichloro l,l,l-éthane. On préfère cependant travailler en l'absence de tout solvant autre que l'acide fluorhydrique.

Pour une meilleure mise en oeuvre de l'invention on préfère travaillier avec un rapport molaire acide nitrique au nombre d'équivalent de radicaux phényle dans l'ester compris entre l et et l,5 et préfentiellement compris entre l et l,2

L'acide fluorhydrique étant utilisé come solvant on peut faire varier le rapport molaire ester de phényle à l'acide fluorhydrique dans de larges limites. D'un point de vue industriel il est cependant préférable de conserver ledit rapport molaire compris entre 5 et 50 et encore plus préférentiellement entre 5 et 20.

L'acide fluorhydrique ayant un point d'ébullition bas sera aisément récupéré par distillation et sera recyclé vers une nouvelle opération laissant un produit réactionnel exempt de solvant.

La température réactionnelle est située notamment entre -20 et l50°C. On préfère cependant lorsque l'on utilise comme matière première le carbonate de biphényle avoir une température réactionnelle comprise entre 20 et l00 et lorsqu'on utilise le phosphate de triphényle avoir une température réactionnelle comprise entre 0°C et 50°C.

La pression réactionnelle peut varier dans de larges limites, par contre lorsque la température réactionnelle est supérieure à 20°C on travaillera de préférence sous une pression supérieure à la pression atmosphérique de façon à conserver l'acide fluorhydrique sous forme liquide.

L'hydrolyse du nitrophényl ester de l'acide minéral a lieu en fonction de la température réactionnelle en même temps que la nitration ou successivement. Lorsque l'on utilise le phosphate de triphényle l'hydrolyse de l'ester nitré a au moins lieu en partie de manière concommittante avec la réaction de nitration. Par contre lorsque l'on part de carbonate de diphényle ; on peut isoler le carbonate de dinitrodiphényle aisément, car il est stable, puis procéder ensuite de manière connue par tout homme de l'art à l'hydrolyse ou procéder à l'hydrolyse directement dans le milieu réactionnel en chauffant.

Lorsque la réaction est terminée on peut éliminer l'acide fluorhydrique par distillation puis récupérer le mélange brut réactionnel à partir duquel est extrait par un solvant tel que le chlorure de méthylène l'ester de nitrophényle recherché.

Le paranitrophénol est utilisé comme intermédiaire de synthèse dans l'industrie pharmaceutique.

La présente invention va être plus complètement décrite à l'aide des exemples suivants qui ne doivent pas être considérés comme limitatifs de l'invention.

EXEMPLE I

Nitration du carbonate de phényle

Dans un réacteur inox de 250 ml agité par barreau aimanté, on introduit sucessivement I00 g (5 moles) d'acide fluorhydrique anhydre, 2I,4g (0,I mole) de carbonate de phényle et I4,5 g (0,22 mole) d'acide nitrique 98 %. Le réacteur est fermé puis porté, sous agitation, à la température de 50°C pendant 3 h 45. Après refroidissement aux environs de 0°C, le réacteur est ouvert et le mélange brut réactionnel coulé sur 200 g de glace pilée. Le mélange hétérogène obtenu est extrait par 2 × I00 cm3 de chlorure de méthylène. Les phases organiques sont rassemblées, lavées par 2 × I00 cm3 d'eau et séchées. Après évaporation, on récupère 26,7 g d'un composé essentiellement constitué de carbonate de nitrophényle (Analyse infrarouge). Après hydrolyse en milieu alcalin et analyse par chromatographie liquide, on observe un rapport isomérique p-nitrophénol/o-nitrophénol d'environ 85/I5.

EXEMPLE 2

Nitration du phosphate de phényle

On opère de maniére analogue (mise en oeuvre, traitements) à l'essai I, avec les composés et conditions suivants :

– HF                       : 100 g (5 moles)

– Phosphate de phényle : 32,6 g (0,1 mole)

– HNO$_3$ 98 %            : 24 g (0,36 mole)

– Durée                   : 2 h. 50 minutes

On récupère 42 g d'un mélange constitué (Analyses par chromatographie liquide haute performance) de :

– Phosphate de phényle      :  0 %

– Phosphate de nitrophényle :  0 %

– Benzoquinone              :  0,2 %

– Phénol                    :  6,9 %

– p–nitrophénol             : 24,4 %

– o–nitrophénol             : 11,8 %

– m–nitrophénol             :  0,1 %

– 2,4 dinitrophénol         : 50,5 %

– Inconnus                  :  6,1 %

EXEMPLE 3

Nitration du phosphate de phényle en solution dans CH$_2$Cl$_2$

On opère de manière analogue (mise en oeuvre) à l,essai I, avec les composés et conditions suivants (le chlorure de méthylène est introduit en premier):

| | |
|---|---|
| – $CH_2Cl_2$ | : 200 cm3 |
| – HF | : 20 g (1 mole) |
| – Phosphate de phényle | : 15 g (0,046 mole) |
| – $HNO_3$ 98 % | : 9,5 g (0,15 mole) |
| – Température | : environ 10°C |
| – Durée | : environ 1 heure |

Après réaction, une partie aliquote (30 cm3) de la phase inférieure qui décante (solution de couleur marron) est traitée par I0 g de NaF, puis filtrée. Par analyse par chromatographie liquide haute performance on met en évidence:

| | |
|---|---|
| – phosphate de phényle | : 5,9 % |
| – phosphate de nitrophényle | : 55,2 % |
| – p–nitrophénol | : 25,9 % |
| – Divers | : 13 % |

Le reste du mélange réactionnel (complément biphasique des 30 cm3 précédent) est traité de manière analogue à l'essai 2. On récupère ainsi I7,5 g d'un composé constitué de (Analyse par chromatographie liquide haute performance)

| | |
|---|---|
| – Inconnus | : 13,6 % |
| – p–nitrophénol | : 70,5 % |
| – o–nitrophénol | : 11,0 % |
| – Phosphate de phényle | : 3 % |

## Revendications

I) Procédé de préparation de paranitrophénol, caractérisé en ce qu'on met en présence dans l'acide fluorhydrique liquide un ester minéral de phényle avec un agent de nitration et en ce qu'on réalise simultanément ou successivement une hydrolyse du produit obtenu.

2) Procédé selon la revendiction I, caractérisé en ce que l'ester minéral de phényle est choisi parmi les carbonates et les phosphates de phénlye.

3) Procédé selon la revendication 2, caractérisé en ce que l'ester minéral de phényle est un phosphate de triphényle.

4) Procédé selon la revendication 2, caractérisé en ce que l'ester minéral de phényle est un carbonate de phényle.

5) Procédé selon la revendication, caractérisé en ce que l'agent de nitration est choisi parmi les acides nitriques présentent une concentration de 65 % minimum, les sels alcalins de l'acide nitrique et les sels de nitronium.

6) Procédé selon la revendication I, caractérisé en ce que l'acide fluorhydrique est anhydre

7) Procédé selon la revendication I, caractérisé en ce que le rapport molaire de l'agent de nitration à l'ester minéral de phényle est compris entre la stoechiométrie et I,5 la stoéchiométrie et de préférence entre la stoéchiométrie et I,2, fois la stoéchiométrie.

8) Procédé selon la revendication I, caractérisé en ce que le rapport molaire de l'ester de phényle à l'HF est compris entre I0 et 50.

9) Procédé selon la revendication I, caractérisé en ce que la température de mise en contact est comprise entre 20°C et I50°C.

0 251 941

I0) Procédé selon les revendications 3 et 9, caractérisé en ce que la température de mise en contact est comprise entre 0°C et 50°C.

II) Procédé selon les revendications 4 et 9, caractérisé en ce que la température de mise en contact est comprise entre 20°C et I00°C.

5

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | FR-A-2 016 335  (FARBENFABRIKEN BAYER) <br> * Revendication 1 * <br><br> ----- | 1 | C 07 C  79/26 <br> C 07 C  69/96 |

DOMAINES TECHNIQUES
RECHERCHES (Int. Cl.4)

C 07 C  79/00
C 07 C  76/00

Le présent rapport de recherche a été établi pour toutes les revendications

| Lieu de la recherche <br> LA HAYE | Date d'achèvement de la recherche <br> 08-09-1987 | Examinateur <br> KLAG M.J. |
|---|---|---|

OEB Form 1503 03 82